# EUROPEAN PATENT APPLICATION

(11) **EP 3 456 739 A1**
(43) Date of publication of application: **20.03.2019**
(21) Application number: 17191990.5
(22) Date of filing: 19.09.2017
(51) Int. Cl.: C07K 16/24, A61P 17/02, A61P 37/00

(54) **USE OF ANTI-TNFALPHA ANTIBODIES FOR TREATING WOUNDS**

(71) Applicant: TILLOTTS PHARMA AG, 4310 Rheinfelden (CH)
(72) Inventor: FURRER, Esther, 4310 Rheinfelden (CH)
(74) Representative: Kalhammer, Georg

(57) **Abstract**

The present invention relates to an antibody comprising a TNFα-binding domain and an Fc region for use in the treatment of wounds.

## Description

### FIELD OF THE INVENTION

The present invention relates to the treatment of wounds. In particular, the invention provides methods of promoting wound healing by administering immunoglobulins comprising a TNFα-binding domain and a functional Fc region.

### BACKGROUND

Wound healing is a vital process characterized by restoration of tissue integrity and homeostasis following e.g. injury. Different immune and non-immune cell types are involved in this process. Macrophages have been shown to play major roles in several aspects that are essential for efficient wound repair. Indeed, macrophages clear invading microbes, contribute to debris scavenging and critically support wound repair by releasing growth factors such as transforming growth factor β, insulin-like growth factor I, hepatocyte growth factor (HGF) and epidermal growth factor. Wound repair depends critically upon macrophages, as depletion during skin injury results in delayed re-epithelialization, reduced collagen deposition, impaired angiogenesis and decreased cell proliferation in the healing wounds. Similarly, re-epithelialization of intestinal ulcers induced by oral administration of dextran sodium sulfate (DSS) is severely delayed by macrophage depletion. Consistently, it has been shown that intestinal healing can be stimulated by the administration of granulocyte-macrophage-colony stimulating factor, a factor that promotes rapid accumulation of macrophages and increases the expression of HGF within the ulcerated mucosa (Bernasconi et al. (2010) Inflamm Bowel Dis.,16, pages 428-441). Using *in-vitro* models of intestinal epithelial repair, evidence has been provided that murine and human macrophages promote epithelial repair through the production of HGF (D'Angelo et al. (2013) Clin Exp Immunol., 174(1), pages 60-72).

There is an ongoing need for methods that promote wound healing and epithelial repair.

### SUMMARY OF THE INVENTION

The inventors of this application found that antibodies to TNFα comprising a functional Fc region induce regulatory macrophages and secretion of HGF. It has further been shown by the inventors that the antibodies are effective in promoting wound healing.

The present invention therefore relates to the subject matter defined in the following items [1] to [43]:
[1] An antibody comprising a TNFα-binding domain and an Fc region for use in the treatment of a wound.
[2] The antibody for use according to item [1], wherein said antibody is an immunoglobulin G (IgG).
[3] The antibody for use according to item [2], wherein said antibody is a class 1 IgG (IgG1).
[4] The antibody for use according to item [1], wherein said antibody comprises an amino acid sequence selected from the group consisting of SEQ ID NO:31, SEQ ID NO:32, SEQ ID NO:33, SEQ ID NO:34 and SEQ ID NO:35.
[5] The antibody for use according to any one of the preceding items, wherein the antibody comprises (i) a V_{L} domain comprising a CDR1 region having the amino acid sequence as shown in SEQ ID NO:1, a CDR2 region having the amino acid sequence as shown in SEQ ID NO:2, and a CDR3 region having the amino acid sequence as shown in SEQ ID NO:3, and (ii) a V_{H} domain comprising a CDR1 region having the amino acid sequence as shown in SEQ ID NO:4, a CDR2 region having the amino acid sequence as shown in SEQ ID NO:5, and a CDR3 region having the amino acid sequence as shown in SEQ ID NO:6.
[6] The antibody for use according to any one of the preceding items, wherein the antibody comprises a V_{H} domain having the amino acid sequence as shown in SEQ ID NO:7and/or a V_{L} domain having the amino acid sequence as shown in SEQ ID NO:8.
[7] The antibody for use according to any one of the preceding items, wherein, the antibody of the invention comprises a light chain having the amino acid sequence as shown in SEQ ID NO:9 and/or a heavy chain having an amino acid sequence selected from the group consisting of SEQ ID NO:10, SEQ ID NO:11, SEQ ID NO:12, SEQ ID NO:13 and SEQ ID NO:14.
[8] The antibody for use according to any one of items [1] to [4], wherein the antibody comprises (i) a V_{L} domain comprising a CDR1 region having the amino acid sequence as shown in SEQ ID NO:15, a CDR2 region having the amino acid sequence as shown in SEQ ID NO:16, and a CDR3 region having the amino acid sequence as shown in SEQ ID NO:17, and (ii) a V_{H} domain comprising a CDR1 region having the amino acid sequence as shown in SEQ ID NO:18, a CDR2 region having the amino acid sequence as shown in SEQ ID NO:19, and a CDR3 region having the amino acid sequence as shown in SEQ ID NO:20.
[9] The antibody for use according to item [8], wherein the antibody comprises a V_{H} domain having the amino acid sequence as shown in SEQ ID NO:21 and/or a V_{L} domain having the amino acid sequence as shown in SEQ ID NO:22 or SEQ ID NO:23.
[10] The antibody for use according to item [8] or [9], wherein the antibody comprises a light chain having the amino acid sequence as shown in SEQ ID NO:24 or SEQ ID NO:25 and/or a heavy chain having an amino acid sequence selected from the group consisting of SEQ ID NO:26, SEQ ID NO:27, SEQ ID NO:28, SEQ ID NO:29 and SEQ ID NO:30.
[11] The antibody for use according to item [1], wherein said antibody is selected from the group consisting of adalimumab, infliximab and golimumab.
[12] The antibody for use according to any one of the preceding items, wherein said antibody is applied topically.
[13] The antibody for use according to any one of the preceding items, wherein said wound affects epithelial cells.
[14] The antibody for use according to any one of the preceding items, wherein epithelial cells are damaged by said wound.
[15] The antibody for use according to any one of the preceding items, wherein said wound is associated with an ulcer in the gastrointestinal tract.
[16] The antibody for use according to any one of the preceding items, wherein said wound is an ulcer in the gastrointestinal tract
[17] The antibody for use according to any one of the preceding items, wherein said wound is associated with a stricturing or penetrating lesion of the intestinal wall.
[18] The antibody for use according to any one of the preceding items, wherein said wound is a stricturing or penetrating lesion of the intestinal wall.
[19] The antibody for use according to any one of items [1] to [14], wherein said wound affects the epidermis.
[20] The antibody for use according to any one of items [1] to [14] and [19], wherein said wound is due to a thermal injury.
[21] The antibody for use according to item [20], wherein said wound includes burns.
[22] The antibody for use according to any one of items [1] to [14], wherein said wound is a chronic wound.
[23] The antibody for use according to item [22], wherein said wound is selected from the group consisting of ulcus cruris, diabetic ulcer, pressure ulcer, and wounds due to tumors.
[24] The antibody for use according to any one of items [1] to [14], wherein said wound is due to a mechanical injury.
[25] The antibody for use according to any one of items [1] to [14], wherein said wound is an iatrogenic wound.
[26] The antibody for use according to item [25], wherein said wound is a surgical wound.
[27] The antibody for use according to any one of items [1] to [14], wherein said wound is associated with fibrosis, necrosis, ischemia, or vasculitis.
[28] The antibody for use according to any one of items [1] to [14], wherein said wound is associated with, or a consequence of inflammatory bowel disease.
[29] The antibody for use according to item [28], wherein said inflammatory bowel disease is ulcerative colitis.
[30] The antibody for use according to item [28] or [29], wherein said inflammatory bowel disease is mild, moderate or severe ulcerative colitis.
[31] The antibody for use according to item [28], wherein said inflammatory bowel disease is Crohn's disease.
[32] The antibody for use according to item [31], wherein said inflammatory bowel disease is mild, moderate or severe Crohn's disease.
[33] The antibody for use according to any one of the preceding items, wherein said antibody is orally administered to a subject.
[34] The antibody for use according to any one of items [1] to [32], wherein said antibody is topically applied to the wound.
[35] The antibody for use according to any one of the preceding items, wherein said antibody is administered to a subject at a dose which is effective to induce regulatory (M2) macrophages.
[36] The antibody for use according to any one of the preceding items, wherein said antibody is administered to a subject at a dose which is effective to induce secretion of HGF in the subject.
[37] The antibody for use according to any one of the preceding items, wherein said treatment comprises administering an anti-inflammatory agent which is different from said antibody.
[38] A method of promoting wound healing, comprising administering to a subject having a wound an effective amount of an antibody comprising a TNFα-binding domain and an Fc region.
[39] A method of enhancing wound healing, comprising administering to a subject having a wound an effective amount of an antibody comprising a TNFα-binding domain and an Fc region.
[40] A method of inducing HGF secretion in a subject, comprising administering to the subject an effective amount of an antibody comprising a TNFα-binding domain and an Fc region.
[41] An antibody comprising a TNFα-binding domain and an Fc region for use in inducing HGF secretion in a subject.
[42] The antibody for use according to item [41], wherein said inducing comprises administering the antibody to the subject.
[43] The antibody for use according to item [41], wherein said antibody is an antibody as defined in any one of items [2] to [11].

### DESCRIPTION OF THE FIGURES

Figure 1: Induction of regulatory (M2) macrophages in a MLR by anti-TNFα antibodies. Only TNFα-specific antibodies with a functional Fc-region induce regulatory (M2) macrophages (54-59%).
Figure 2: CD206 expression on CD14⁺ cells following anti-TNFα treatment. TNFα-specific antibodies containing a fully functional Fc region (clone cA2, adalimumab and TP-IgG) express significantly higher levels of CD206 as shown by MFI. *: p<0.05 (n=4)
Figure 3: Wound healing scratch assay. Percent closure compared to t=0. *: p<0.05 compared with control IgG.
Figure 4: Wound healing scratch assay. Stimulation index is the BrdU uptake ratio compared to control IgG. **: p<0.01 compared with control IgG.
Figure 5: HGF concentration in supernatant of MLRs treated with anti-TNF-α antibodies. Only TNFα specific antibodies with a fully functional Fc-region induce the secretion of HGF. BLQ: below limit of quantitation.
Figure 6: HGF protein expression in *ex vivo* colon tissue collected from Crohn's disease patients after treatment with TNFα antagonists (10 µg/mL; n = 3).

### DETAILED DESCRIPTION

The present invention relates to an antibody comprising a TNFα-binding domain and an Fc region for use in the treatment of a wound.

The antibody of the invention comprises a domain that is capable of binding to TNFα and a functional Fc region.

In the context of the present application, the term "antibody" is used as a synonym for "immunoglobulin" (Ig), which is defined as a protein belonging to the class IgG, IgM, IgE, IgA, or IgD (or any subclass thereof), and includes all conventionally known antibodies and functional fragments thereof. In the context of the present invention, a "functional fragment" of an antibody/immunoglobulin is defined as antigen-binding fragment or other derivative of a parental antibody that essentially maintains the ability to bind to the antigen and still comprises a functional Fc domain. The antibodies of the present invention may be part of bi-or multifunctional constructs.

Preferably the antibody is a monoclonal antibody. The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced. Monoclonal antibodies can be prepared using a wide variety of techniques known in the art including the use of hybridoma, recombinant, and phage display technologies, or a combination thereof. (Harlow and Lane, "Antibodies, A Laboratory Manual" CSH Press 1988, Cold Spring Harbor N.Y.).

In other embodiments, including embodiments relating to the *in vivo* use of the anti-TNFα antibodies in humans, chimeric, primatized, humanized, or human antibodies can be used. In a preferred embodiment, the antibody is a human antibody or a humanized antibody, more preferably a monoclonal human antibody or a monoclonal humanized antibody.

In another particular embodiment the antibody of the invention is an immunoglobulin, preferably an immunoglobulin G (IgG). The subclass of the IgG of the invention is not limited and includes IgG₁, IgG₂, IgG₃, and IgG₄. Preferably, the IgG of the invention is of subclass 1, 2 or 4, i.e. it is an IgG₁, IgG₂, or IgG₄ molecule, respectively. Most preferably, the IgG of the invention is of subclass 1, i.e. it is an IgG₁ molecule.

### Antibody and TNFα-binding domain

The TNFα-binding domain of the antibody of the invention is not particularly limited. It can be derived from any antibody that is capable of binding to TNFα.

Preferably, the antibody of the invention specifically binds to TNFα. As used herein, an antibody "specifically recognizes", or "specifically binds to" human TNFα, when the antibody is able to discriminate between human TNFα and one or more reference molecule(s). Preferably, the IC₅₀ value for binding to each of the reference molecules is at least 1,000 times greater than the IC₅₀ value for binding to TNFα. In its most general form (and when no defined reference is mentioned), "specific binding" is referring to the ability of the antibody to discriminate between human TNFα and an unrelated biomolecule, as determined, for example, in accordance with a specificity assay methods known in the art. Such methods comprise, but are not limited to, Western blots and ELISA tests. For example, a standard ELISA assay can be carried out. Typically, determination of binding specificity is performed by using not a single reference biomolecule, but a set of about three to five unrelated biomolecules, such as milk powder, BSA, transferrin or the like. In one embodiment, specific binding refers to the ability of the antibody to discriminate between human TNFα and human TNFβ.

The antibody of the invention preferably has a high affinity to human TNFα. The term "K_{D}," refers to the dissociation equilibrium constant of a particular antibody-antigen interaction. Typically, the antibody of the invention binds to human TNFα with a dissociation equilibrium constant (K_{D}) of less than approximately 2x10⁻¹⁰ M, preferably less than 1.5x10⁻¹⁰ M, preferably less than 1.25x10⁻¹⁰ M, more preferably less than 1x10⁻¹⁰ M, most preferably less than 7.5x10⁻¹¹ M or even less than 5x10⁻¹¹ M, as determined using surface plasmon resonance (SPR) technology in a BIACORE instrument.

Several monoclonal antibodies against TNFα have been described in the prior art. In WO 92/11383, recombinant antibodies, including CDR-grafted antibodies, specific for TNFα are disclosed. U.S. Pat No. 5,919,452 discloses anti-TNFα chimeric antibodies and their use in treating pathologies associated with the presence of TNFα. Further anti-TNFα antibodies are disclosed in Stephens et al. (Immunology, 85, 668-674, 1995), GB-A-2 246 570, GB-A-2 297 145, US 8,673,310, US 2014/0193400, EP 2 390 267 B1, US 8,293,235, US 8,697,074, WO 2009/155723 A2 and WO 2006/131013 A2.

In one embodiment, the antibody of the invention is selected from the group consisting of infliximab, adalimumab, golimumab, and biosimilar versions thereof.

In another embodiment of the present invention, the antibody of the invention is an anti-TNFα antibody as disclosed in any one of PCT applications PCT/EP2017/056218, PCT/EP2017/056246, PCT/EP2017/056237 and PCT/EP2017/056227 as originally filed. In yet another embodiment of the present invention, the antibody is an anti-TNFα antibody with a light chain variable domain and/or a heavy chain variable domain comprising complementarity-determining regions (CDRs) with amino acid sequences as disclosed in PCT applications PCT/EP2017/056218, PCT/EP2017/056246, PCT/EP2017/056237 and PCT/EP2017/056227 as originally filed.

In a preferred embodiment of the present invention, the antibody is an anti-TNFα antibody with a light chain variable domain and/or a heavy chain variable domain comprising one or more CDRs with amino acid sequences as disclosed in SEQ ID NO:7, 9, 12, 14, 24 and 25 of PCT/EP2017/056218, in SEQ ID NO:7-11 and 6 of PCT/EP2017/056246, in SEQ ID NO:7-12 of PCT/EP2017/056237, in SEQ ID NO:1-4, 7 and 6 of PCT/EP2017/056227, and combinations thereof. In another preferred embodiment of the present invention, the antibody is an anti-TNFα antibody with a light chain variable domain and a heavy chain variable domain comprising CDRs with amino acid sequences as disclosed in claim 2 of PCT/EP2017/056218, in claim 2 of PCT/EP2017/056246, in claim 2 of PCT/EP2017/056237 or in claim 2 PCT/EP2017/056227, as originally filed. In yet another preferred embodiment of the present invention, the anti-TNFα antibody is selected from the group consisting of anti-TNFα antibodies comprising a heavy chain variable domain amino acid sequence and/or a light chain variable domain amino acid sequence according to claim 4 of PCT/EP2017/056218, claims 5 and 6 of PCT/EP2017/056246, claims 5 and 6 of PCT/EP2017/056237, claim 4 of PCT/EP2017/056227, and combinations thereof. The disclosure of each of the international patent applications PCT/EP2017/056218, PCT/EP2017/056246, PCT/EP2017/056237 and PCT/EP2017/056227 is incorporated herein in its entirety. They form part of the disclosure of the present application.

Throughout the present specification and claims, the Kabat numbering system is generally used when referring to a residue in the variable domain (approximately, residues 1-107 of the light chain and residues 1-113 of the heavy chain) (Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat et al., Sequences of Proteins of Immunological Interest, 5th Ed. Public Health Service, National Institutes of Health, Bethesda, Md. (1991) expressly incorporated herein by reference). Unless stated otherwise herein, references to residues numbers in the variable domain of antibodies means residue numbering by the Kabat numbering system. Unless stated otherwise herein, references to residue numbers in the constant domain of antibodies means residue numbering by the EU numbering system (see e.g., WO 2006/073941).

In a particular embodiment, the antibody of the invention comprises a heavy chain which comprises an Fc region and a hinge region, wherein said "Fc region and hinge region" comprises or consists of an amino acid sequence selected from the group consisting of SEQ ID NO:31, SEQ ID NO:32, SEQ ID NO:33, SEQ ID NO:34 and SEQ ID NO:35.

In another particular embodiment, the antibody of the invention comprises (i) a V_{L} domain comprising a CDR1 region having the amino acid sequence as shown in SEQ ID NO:1, a CDR2 region having the amino acid sequence as shown in SEQ ID NO:2, and a CDR3 region having the amino acid sequence as shown in SEQ ID NO:3, and (ii) a V_{H} domain comprising a CDR1 region having the amino acid sequence as shown in SEQ ID NO:4, a CDR2 region having the amino acid sequence as shown in SEQ ID NO:5, and a CDR3 region having the amino acid sequence as shown in SEQ ID NO:6.

In a more preferred embodiment, the antibody of the invention comprises a V_{H} domain having the amino acid sequence as shown in SEQ ID NO:7. In another more preferred embodiment the antibody comprises a V_{L} domain having the amino acid sequence as shown in SEQ ID NO:8. Most preferably, the antibody of the invention comprises (i) a V_{H} domain having the amino acid sequence as shown in SEQ ID NO:7, and (ii) a V_{L} domain having the amino acid sequence as shown in SEQ ID NO:8.

Most preferably, the antibody of the invention comprises a light chain having the amino acid sequence as shown in SEQ ID NO:9 and/or a heavy chain having an amino acid sequence selected from the group consisting of SEQ ID NO:10, SEQ ID NO:11, SEQ ID NO:12, SEQ ID NO:13 and SEQ ID NO:14.

In yet another another particular embodiment, the antibody of the invention comprises (i) a V_{L} domain comprising a CDR1 region having the amino acid sequence as shown in SEQ ID NO:15, a CDR2 region having the amino acid sequence as shown in SEQ ID NO:16, and a CDR3 region having the amino acid sequence as shown in SEQ ID NO:17, and (ii) a V_{H} domain comprising a CDR1 region having the amino acid sequence as shown in SEQ ID NO:18, a CDR2 region having the amino acid sequence as shown in SEQ ID NO:19, and a CDR3 region having the amino acid sequence as shown in SEQ ID NO:20.

In a more preferred embodiment, the antibody of the invention comprises a V_{H} domain having the amino acid sequence as shown in SEQ ID NO:21. In another more preferred embodiment the antibody comprises a V_{L} domain having the amino acid sequence as shown in SEQ ID NO:22 or SEQ ID NO:23. Most preferably, the antibody of the invention comprises (i) a V_{H} domain having the amino acid sequence as shown in SEQ ID NO:21, and (ii) a V_{L} domain having the amino acid sequence as shown in SEQ ID NO:22 or SEQ ID NO:23.

Most preferably, the antibody of the invention comprises a light chain having the amino acid sequence as shown in SEQ ID NO:24 or SEQ ID NO:25 and/or a heavy chain having an amino acid sequence selected from the group consisting of SEQ ID NO:26, SEQ ID NO:27, SEQ ID NO:28, SEQ ID NO:29 and SEQ ID NO:30.

In a preferred aspect of the invention, the antibody of the invention is a non-fucosylated antibody or an antibody having reduced fucosylation. The term "antibody having reduced fucosylation", as used herein, refers to an antibody in which less than 90% of the N-glycans of the antibody are fucosylated. Methods to determine the percentage of fucosylation are known in the art. In one embodiment, less than 75%, or less than 50%, or less than 25% of the N-glycans of the antibody are fucosylated. Most preferably, less than 15%, or less than 10%, of the N-glycans of the antibody are fucosylated. In a particular embodiment, the N-glycans of the antibody of the invention do not contain any fucose. Preferably, less than 90% of the N-glycans at N297 (EU numbering) of the antibody are fucosylated. In another embodiment, less than 75%, or less than 50%, or less than 25% of the N-glycans at N297 (EU numbering) of the antibody are fucosylated. Most preferably, less than 15%, or less than 10%, of the N-glycans at N297 (EU numbering) of the antibody are fucosylated. In another embodiment, the N-glycans at N297 of the antibody do not contain any fucose.

Non-fucosylated antibodies, sometimes also referred to as afucosylated antibodies, can be generated by various methods. For example, the synergistic knockdown of the genes for α1,6-fucosyltransferase (*FUT8*) and GDP-mannose 4,6-dehydratase (*GMD*) in CHO cells can be used to produce monoclonal antibody variants that are fully afucosylated and ADCC-enhanced (see, e.g., Imai-Nishiya et al. (2007) BMC Biotechnol. 7, 84). A method using zinc-finger nucleases (ZFNs) cleaving the *FUT8* gene in a region encoding the catalytic core of the α1,6-fucosyltransferase and thus disrupting the corresponding enzymatic function in CHO cells can be used to produce monoclonal antibodies completely lacking core fucose (see, e.g., Malphettes et al. (2010) Biotechnol. Bioeng. 106, 774-783).

Antibodies having reduced fucosylation can be prepared by addition of a decoy substrate such as 2-deoxy-2-fluoro-2-fucose to the culture medium (see, e.g., Dekker et al. (2016) Sci Rep 6:36964), resulting in a reduced incorporation of fucose in the IgG-Fc glycans.

In another embodiment, the antibody of the invention has a high sialic acid content. In increase in sialylation can be achieved, e.g. by simultaneous transfection of cytidine monophosphate-sialic acid synthase (CMP-SAS), cytidine monophosphate-sialic acid transporter (CMP-SAT), and α 2,3-sialyltransferases (see, e.g., Son et al. (2011) Glycobiology 21, 1019-1028).

Preferably, the affinity at pH 6 to human FcRn of the antibody of the invention is high. The high affinity binding of the antibody to human FcRn at pH 6 is characterized by a K_{D} value of less than 500 nM. Preferably, the K_{D} value of the high affinity binding at pH 6 is less than 400 nM, or less than 300 nM, or less than 200 nM. For example, the K_{D} value characterizing the affinity at pH 6 may be in the range from 1 to 500 nM, or 2 to 400 nM, or 3 to 300 nM, or 4 to 200 nM, or 5 to 100 nM.

In one embodiment, the affinity of the antibody of the invention to human FcRn at pH 6 is greater than the affinity of infliximab to human FcRn at pH 6.0.

The affinity of the antibody of the invention to human FcRn is preferably determined by surface plasma resonance (SPR).

The antibody of the present invention typically has a low affinity to human FcRn at pH 7.4. The low affinity is characterized by a K_{D} value of greater than 1 µM. Preferably, the low affinity to human FcRn at pH 7.4 is characterized by a K_{D} value of greater than 2 µM, or greater than 5 µM, or greater than 10 µM.

In a particular embodiment, the low affinity at pH 7.4 is so low that a K_{D} value cannot be determined by SPR.

In a special embodiment, the ratio of (i) a K_{D} value for binding of the antibody of the invention to human FcRn at pH 7.4 to (ii) a K_{D} value for binding to human FcRn at pH 6.0, is at least 50. Preferably, this ratio is at least 100, or at least 150, or at least 200.

In a specific aspect of the invention, the antibody of the invention is efficiently transported across a polarized cell monolayer from the apical side to the basolateral side. The polarized cell monolayer may be a monolayer of polarized T84 cells. Preferably, the transport across the polarized cell monolayer is in a greater amount than that of infliximab, wherein the amount of antibody in infliximab refers to the mass/cm² of the polarized cell monolayer. The amount of antibody transported across the polarized cell monolayer, relative to the amount of infliximab transported across the polarized cell monolayer, is preferably at least 110%, or at least 120%, more preferably at least 130%, or at least 140%, or at least 150% (wherein the amount of transported infliximab is set to 100%).

In a further embodiment, the antibody is specifically transported across the polarized cell monolayer from the apical side to the basolateral side in the presence of an excess of competing immunoglobulins. This is referred to as specific transport herein.

The percentage of the total mass of immunoglobulins transported across the polarized cell monolayer is preferably greater than the percentage of infliximab transported across the polarized cell monolayer from the apical side to the basolateral side in the presence of a 10-fold excess of competing immunoglobulins. The percentage of antibody of the invention transported across the polarized cell monolayer in the presence of a 10-fold excess of unrelated immunoglobulins, relative to the percentage of infliximab transported across the polarized cell monolayer in the presence of a 10-fold excess of unrelated antibodies, is preferably at least 120%, or at least 130%, or at least 140%, or at least 150% (infliximab is set to be 100%).

In other aspects of the invention, the antibody of the invention is further capable of inducing CD14⁺CD206⁺ macrophages. The level of induction is preferably comparable to, equal to, or greater than that of infliximab. The antibody of the invention may further be capable of suppressing T cell proliferation. The degree of suppression of T cell proliferation is preferably comparable to, equal to, or greater than that of infliximab.

In a particular embodiment, the antibody of the invention is more resistant to proteolytic degradation than a wildtype antibody (e.g. infliximab). This applies, e.g. to proteolytic degradation by matrix metalloproteinase 3 (MMP-3), immunoglobulin G-degrading enzyme of S. pyogenes (IdeS), Endoproteinase Glu-C from Staphylococcus aureus strain V8 (GluC), or combinations thereof.

### Pharmaceutical Compositions and Treatment

Treatment of a disease encompasses the treatment of patients already diagnosed as having any form of the disease at any clinical stage or manifestation; the delay of the onset or evolution or aggravation or deterioration of the symptoms or signs of the disease; and/or preventing and/or reducing the severity of the disease.

A "subject" or "patient" to whom an anti-TNFα antibody is administered can be a mammal, such as a non-primate (e.g., cow, pig, horse, cat, dog, rat, etc.) or a primate (e.g., monkey or human). In certain aspects, the human is a pediatric patient. In other aspects, the human is an adult patient.

Compositions comprising an anti-TNFα antibody and, optionally one or more additional therapeutic agents, such as the second therapeutic agents described below, are described herein. The compositions typically are supplied as part of a sterile, pharmaceutical composition that includes a pharmaceutically acceptable carrier. This composition can be in any suitable form (depending upon the desired method of administering it to a patient).

The anti-TNFα antibodies can be administered to a patient by a variety of routes such as orally, transdermally, subcutaneously, intranasally, intravenously, intramuscularly, intrathecally, topically, mucosally or locally. The most suitable route for administration in any given case will depend on the particular antibody, the subject, and the nature and severity of the disease and the physical condition of the subject. In one embodiment, the anti-TNFα antibody is administered intravenously.

The anti-TNFα antibody may be present in a pharmaceutical composition at a concentration sufficient to permit intravenous administration at 0.5 mg/kg body weight to 20 mg/kg body weight. In some embodiments, the concentration of antibody suitable for use in the compositions and methods described herein includes, but is not limited to, 0.5 mg/kg, 0.75 mg/kg, 1 mg/kg, 2 mg/kg, 2.5 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg, or a concentration ranging between any of the foregoing values, e.g., 1 mg/kg to 10 mg/kg, 5 mg/kg to 15 mg/kg, or 10 mg/kg to 18 mg/kg.

The effective dose of an anti-TNFα antibody can range from about 0.001 to about 750 mg/kg per single (e.g., bolus) administration, multiple administrations or continuous administration, or to achieve a serum concentration of 0.01-5000 µg/ml serum concentration per single (e.g., bolus) administration, multiple administrations or continuous administration, or any effective range or value therein depending on the condition being treated, the route of administration and the age, weight and condition of the subject. In case of oral administration, the serum concentration may be very low or even below the detection limit. In certain embodiments, each dose can range from about 0.5 mg to about 50 mg per kilogram of body weight or from about 3 mg to about 30 mg per kilogram body weight. The antibody can be formulated as an aqueous solution.

In one embodiment, the antibody of the invention is administered orally. If the administration is via the oral route the antibody is preferably an IgG, most preferably an IgG₁. If the antibody is administered orally, the daily dose of antibody is typically in the range of about 0.01 mg/kg to about 100 mg/kg of body weight, or about 0.05 mg/kg to about 50 mg/kg of body weight, or about 0.1 mg/kg to about 25 mg/kg of body weight, or about 0.15 mg/kg to about 10 mg/kg of body weight, or about 0.16 mg/kg to about 5 mg/kg of body weight, or about 0.2 mg/kg to about 2 mg/kg of body weight, or about 0.2 mg/kg to about 1 mg/kg of body weight, Generally, advantageous doses are doses of 1 to 200 mg per day, preferably 5 to 100 or 10 to 50 mg per day.

Pharmaceutical compositions can be conveniently presented in unit dose forms containing a predetermined amount of an anti-TNFα antibody per dose. Such a unit can contain 0.5 mg to 5 g, for example, but without limitation, 1 mg, 10 mg, 20 mg, 30 mg, 40 mg, 50 mg, 100 mg, 200 mg, 300 mg, 400 mg, 500 mg, 750 mg, 1000 mg, or any range between any two of the foregoing values, for example 10 mg to 1000 mg, 20 mg to 50 mg, or 30 mg to 300 mg. Pharmaceutically acceptable carriers can take a wide variety of forms depending, e.g., on the condition to be treated or route of administration.

Determination of the effective dosage, total number of doses, and length of treatment an anti-TNFα antibody thereof is well within the capabilities of those skilled in the art, and can be determined using a standard dose escalation study.

Therapeutic formulations of the anti-TNFα antibodies suitable in the methods described herein can be prepared for storage as lyophilized formulations or aqueous solutions by mixing the antibody having the desired degree of purity with optional pharmaceutically-acceptable carriers, excipients or stabilizers typically employed in the art (all of which are referred to herein as "carriers"), i.e., buffering agents, stabilizing agents, preservatives, isotonifiers, non-ionic detergents, antioxidants, and other miscellaneous additives. See, Remington's Pharmaceutical Sciences, 16th edition (Osol, ed. 1980). Such additives must be nontoxic to the recipients at the dosages and concentrations employed.

Buffering agents help to maintain the pH in the range which approximates physiological conditions. They can present at concentration ranging from about 2 mM to about 50 mM. Suitable buffering agents include both organic and inorganic acids and salts thereof such as citrate buffers (e.g., monosodium citrate-disodium citrate mixture, citric acid-trisodium citrate mixture, citric acid-monosodium citrate mixture, etc.), citrate-phosphate buffers, succinate buffers (e.g., succinic acid- monosodium succinate mixture, succinic acid-sodium hydroxide mixture, succinic acid- disodium succinate mixture, etc.), tartrate buffers (e.g., tartaric acid-sodium tartrate mixture, tartaric acid-potassium tartrate mixture, tartaric acid-sodium hydroxide mixture, etc.), fumarate buffers (e.g., fumaric acid-monosodium fumarate mixture, fumaric acid-disodium fumarate mixture, monosodium fumarate-disodium fumarate mixture, etc.), gluconate buffers (e.g., gluconic acid-sodium glyconate mixture, gluconic acid-sodium hydroxide mixture, gluconic acid-potassium glyuconate mixture, etc.), oxalate buffer (e.g., oxalic acid-sodium oxalate mixture, oxalic acid-sodium hydroxide mixture, oxalic acid-potassium oxalate mixture, etc), lactate buffers (e.g., lactic acid-sodium lactate mixture, lactic acid-sodium hydroxide mixture, lactic acid-potassium lactate mixture, etc.) and acetate buffers (e.g., acetic acid-sodium acetate mixture, acetic acid-sodium hydroxide mixture, etc.). Additionally, phosphate buffers, histidine buffers and trimethylamine salts such as Tris can be used.

The pharmaceutical composition of the invention may further comprise at least one salt, e.g. sodium chloride. The salt concentration preferably ranges from 100 mM to 200 mM, e.g. about 150 mM.

Preservatives can be added to retard microbial growth, and can be added in amounts ranging from 0.2%- 1% (w/v). Suitable preservatives include phenol, benzyl alcohol, metacresol, methyl paraben, propyl paraben, octadecyldimethylbenzyl ammonium chloride, benzalconium halides (e.g., chloride, bromide, and iodide), hexamethonium chloride, and alkyl parabens such as methyl or propyl paraben, catechol, resorcinol, cyclohexanol, and 3-pentanol. Isotonifiers sometimes known as "stabilizers" can be added to ensure isotonicity of liquid compositions and include polyhydric sugar alcohols, preferably trihydric or higher sugar alcohols, such as glycerin, erythritol, arabitol, xylitol, sorbitol and mannitol. Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, etc., organic sugars or sugar alcohols, such as lactose, trehalose, stachyose, mannitol, sorbitol, xylitol, ribitol, myoinositol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (e.g., peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran. Stabilizers can be present in the range from 0.1 to 10,000 weights per part of weight active protein.

Non-ionic surfactants or detergents (also known as "wetting agents") can be added to help solubilize the therapeutic agent as well as to protect the therapeutic protein against agitation-induced aggregation, which also permits the formulation to be exposed to shear surface stressed without causing denaturation of the protein. Suitable non-ionic surfactants include polysorbates (20, 80, etc.), polyoxamers (184, 188 etc.), Pluronic polyols, polyoxyethylene sorbitan monoethers (TWEEN®-20, TWEEN®-80, etc.). Non-ionic surfactants can be present in a range of about 0.05 mg/ml to about 1.0 mg/ml, or in a range of about 0.07 mg/ml to about 0.2 mg/ml.

Additional miscellaneous excipients include bulking agents (e.g., starch), chelating agents (e.g., EDTA), antioxidants (e.g., ascorbic acid, methionine, vitamin E), protease inhibitors and co-solvents.

The formulation herein can also contain a second therapeutic agent in addition to an anti-TNFα antibody thereof. Examples of suitable second therapeutic agents are provided below.

The dosing schedule can vary from once a month to daily depending on a number of clinical factors, including the type of disease, severity of disease, and the patient's sensitivity to the anti-TNFα antibody. In specific embodiments, an anti-TNFα antibody thereof is administered daily, twice weekly, three times a week, every other day, every 5 days, once weekly, every 10 days, every two weeks, every three weeks, every four weeks or once a month, or in any range between any two of the foregoing values, for example from every four days to every month, from every 10 days to every two weeks, or from two to three times a week, etc.

The dosage of an anti-TNFα antibody to be administered will vary according to the particular antibody, the subject, and the nature and severity of the disease, the physical condition of the subject, the therapeutic regimen (e.g., whether a second therapeutic agent is used), and the selected route of administration; the appropriate dosage can be readily determined by a person skilled in the art.

It will be recognized by one of skill in the art that the optimal quantity and spacing of individual dosages of an anti-TNFα antibody thereof will be determined by the nature and extent of the condition being treated, the form, route and site of administration, and the age and condition of the particular subject being treated, and that a physician will ultimately determine appropriate dosages to be used. This dosage can be repeated as often as appropriate. If side effects develop the amount and/or frequency of the dosage can be altered or reduced, in accordance with normal clinical practice.

### Wounds and disorders to be treated

The invention relates to a method of treating wounds in a subject, comprising administering to the subject the antibody as defined herein. The wound may be a chronic wound, e.g. ulcus cruris, diabetic ulcer, pressure ulcer, or a wound due to a tumor.

In another embodiment, the wound affects epithelial cells, which may be damaged by the wound.

In other embodiments the wound to be treated is associated with an ulcer in the gastrointestinal tract. Such a wound may be associated with a stricturing or penetrating lesion of the intestinal wall.

In yet another embodiment, the wound affects the epidermis.

In yet another embodiment, the wound is due to a thermal injury. The wound may include burns.

In yet another embodiment, the wound is due to a mechanical injury.

In yet another embodiment, the wound is an iatrogenic wound, e.g. a surgical wound.

In a particular embodiment, the wound is not associated with inflammation. In another particular embodiment, the wound is not an inflammatory wound.

In another embodiment, the wound is associated with necrosis, ischemia or vasculitis, e.g. in the case of gangrene.

In another embodiment, the wound is associated with fibrosis and/or necrosis.

In other embodiments the wound is associated with, or due to, or the consequence of, any one of the following disorders:
Chronic and/or autoimmune states of inflammation in general, immune mediated inflammatory disorders in general, inflammatory CNS disease, inflammatory diseases affecting the eye, joint, skin, mucous membranes, central nervous system, gastrointestinal tract, urinary tract or lung, states of uveitis in general, retinitis, HLA-B27+ uveitis, Behçet's disease, dry eye syndrome, glaucoma, Sjögren syndrome, diabetes mellitus (incl. diabetic neuropathy), insulin resistance, states of arthritis in general, rheumatoid arthritis, osteoarthritis, reactive arthritis and Reiter's syndrome, juvenile arthritis, ankylosing spondylitis, multiple sclerosis, Guillain-Barré syndrome, myasthenia gravis, amyotrophic lateral sclerosis, sarcoidosis, glomerulonephritis, chronic kidney disease, cystitis, psoriasis (incl. psoriatic arthritis), hidradenitis suppurativa, panniculitis, pyoderma gangrenosum, SAPHO syndrome (synovitis, acne, pustulosis, hyperostosis and osteitis), acne, Sweet's syndrome, pemphigus, Crohn's disease (incl. extraintestinal manifestastations), ulcerative colitis, asthma bronchiale, hypersensitivity pneumonitis, general allergies, allergic rhinitis, allergic sinusitis, chronic obstructive pulmonary disease (COPD), lung fibrosis, Wegener's granulomatosis, Kawasaki syndrome, Giant cell arteritis, Churg-Strauss vasculitis, polyarteritis nodosa, burns, graft versus host disease, host versus graft reactions, rejection episodes following organ or bone marrow transplantation, systemic and local states of vasculitis in general, systemic and cutaneous lupus erythematodes, polymyositis and dermatomyositis, sclerodermia, pre-eclampsia, acute and chronic pancreatitis, viral hepatitis, alcoholic hepatitis, postsurgical inflammation such as after eye surgery (e.g. cataract (eye lens replacement) or glaucoma surgery), joint surgery (incl. arthroscopic surgery), surgery at joint-related structures (e.g. ligaments), oral and/or dental surgery, minimally invasive cardiovascular procedures (e.g. PTCA, atherectomy, stent placement), laparoscopic and/or endoscopic intra-abdominal and gynecological procedures, endoscopic urological procedures (e.g. prostate surgery, ureteroscopy, cystoscopy, interstitial cystitis), or perioperative inflammation (prevention) in general, bullous dermatitis, neutrophilic dermatitis, toxic epidermal necrolysis, pustular dermatitis, cerebral malaria, hemolytic uremic syndrome, allograft rejection, otitis media, snakebite, erythema nodosum, myelodysplastic syndromes, primary sclerosing cholangitis, seronegative spondyloartheropathy, autoimmune hematolytic anemia, orofacial granulamatosis, pyostomatitis vegetans, aphthous stomatitis, geographic tongue, migratory stomatitis, Alzheimer's disease, Parkinson's disease, Huntington's disease, Bell's palsy, Creutzfeld-Jakob disease and neuro-degenerative conditions in general; cancer-related osteolysis, cancer-related inflammation, cancer-related pain, cancer-related cachexia, bone metastases, acute and chronic forms of pain, sciatica, low back pain, carpal tunnel syndrome, complex regional pain syndrome (CRPS), gout, postherpetic neuralgia, fibromyalgia, local pain states, chronic pain syndroms due to metastatic tumor, dysmenorrhea; states of arthritis in general, rheumatoid arthritis, osteoarthritis, reactive arthritis, juvenile arthritis; psoriasis incl. psoriatic arthritis; inflammatory bowel disease, including Crohn's disease, ulcerative colitis incl. proctitis, sigmoiditis, left-sided colitis, extensive colitis and pancolitis, undetermined colitis, microscopic colitis incl. collagenous and lymphocytic colitis, colitis in connective tissue disease, diversion colitis, colitis in diverticular disease, eosinophilic colitis and pouchitis.

Most preferably, the antibody of the invention is used to treat wounds that are associated with, or due to, or a consequence of, an inflammatory bowel disease, in particular Crohn's disease, ulcerative colitis or microscopic colitis. The Crohn's disease may be ileal, colonic, ileocolonic or isolated upper Crohn's disease (gastric, duodenal and/or jejunal) including non-stricturing/non-penetrating, stricturing, penetrating and perianal disease behavior, allowing any combination of localization and disease behavior of any of the above mentioned. The ulcerative colitis may be ulcerative proctitis, sigmoiditis, proctosigmoiditis, left-sided colitis, pan-ulcerative colitis and pouchitis.

In a preferred embodiment of the invention, the antibody is administered topically to the wound.

Topical compositions can be in the form of liquids, ointments or creams, dry powders, liquid-based aerosols, or suppositories (e.g. carbowax or glycerin preparations, which may be used for rectal or vaginal administration of the antibody) or rectal enemas. Alternatively, the antibody may be administered in the form of a wound dressing or a patch.

### Combination Therapy and other aspects

Preferably, the patient being treated with an anti-TNFα antibody is also treated with another conventional medicament. For example, a patient suffering from inflammatory bowel disease, especially if having moderate to severe disease is typically also being treated with mesalazine or derivatives or prodrugs thereof, corticosteroids, e.g. budesonide or prednisolone (oral or i.v.), immunosuppressants, e.g. azathioprine/6-mercaptopurine (6-MP) or methotrexate, cyclosporine or tacrolimus. Other medicaments which can be co-administered to the patient include other anti-TNFα antibodies (e.g. infliximab, adalimumab, etanercept, certolizumab pegol, golimuamb), integrin antagonists (e.g. natalizumab, vedolizumab), anti-IL-23 antibodies (e.g. MEDI2070), anti-β7 antibodies (e.g. etrolizumab), JAK inhibitors in the JAK/STAT pathway (e.g. tofacitinib), and others. Further medicaments which can be co-administered to the patient include immunosuppressants (e.g. azathioprine/6-MP or methotrexate or oral cyclosporine) in order to maintain stable and longer remission. Yet another aspect of the invention is the use of an anti-TNFα antibody as defined hereinabove for reducing inflammation.

Another aspect of the invention is a method of promoting wound healing, comprising administering to a subject having a wound an effective amount of an antibody comprising a TNFα-binding domain and an Fc region. The antibody to be used according to this aspect is preferably an antibody as defined herein.

Another aspect of the invention is a method of enhancing wound healing, comprising administering to a subject having a wound an effective amount of an antibody comprising a TNFα-binding domain and an Fc region. The antibody to be used according to this aspect is preferably an antibody as defined herein.

Another aspect of the invention is a method of inducing HGF secretion in a subject, comprising administering to the subject an effective amount of an antibody comprising a TNFα-binding domain and an Fc region. The antibody to be used according to this aspect is preferably an antibody as defined herein.

Another aspect of the invention is an antibody comprising a TNFα-binding domain and an Fc region for use in inducing HGF secretion in a subject. This preferably includes administering the antibody to the subject. The antibody to be used according to this aspect is preferably an antibody as defined herein.

**Table 1. Overview of the sequences of the sequence listing.**

| **SEQ ID NO:** | **Description of the amino acid sequence** |
|---|---|
| 1 | CDR L1 of clone 16-22-H05 |
| 2 | CDR L2 of clone 16-22-H05 |
| 3 | CDR L3 of clone 16-22-H05 |
| 4 | CDR H1 of clone 16-22-H05 |
| 5 | CDR H2 of clone 16-22-H05 |
| 6 | CDR H3 of clone 16-22-H05 |
| 7 | V_{H} of humanized IgG of clone 16-22-H05 |
| 8 | V_{L} of humanized IgG of clone 16-22-H05 |
| 9 | Light chain of humanized IgG of clone 16-22-H05 |
| 10 | Heavy chain of Ab-YTE-DLE-PVAΔG (based on clone 16-22-H05) |
| 11 | Heavy chain of Ab-A-DLE-PVAΔG (based on clone 16-22-H05) |
| 12 | Heavy chain of Ab-A-AEA-PVAΔG (based on clone 16-22-H05) |
| 13 | Heavy chain of Ab-AA (based on clone 16-22-H05) |
| 14 | Heavy chain of Ab-REW (based on clone 16-22-H05) |
| 15 | CDR L1 of clone 17-22-B03 |
| 16 | CDR L2 of clone 17-22-B03 |
| 17 | CDR L3 of clone 17-22-B03 |
| 18 | CDR H1 of clone 17-22-B03 |
| 19 | CDR H2 of clone 17-22-B03 |
| 20 | CDR H3 of clone 17-22-B03 |
| 21 | V_{H} of humanized IgG of clone 17-22-B03 |
| 22 | V_{L} of humanized IgG of clone 17-22-B03 (sc08) |
| 23 | V_{L} of humanized IgG of clone 17-22-B03 (sc02) |
| 24 | Light chain of humanized IgG of clone 17-22-B03 (sc08) |
| 25 | Light chain of humanized IgG of clone 17-22-B03 (sc02) |
| 26 | Heavy chain of Ab-YTE-DLE-PVAΔG (based on clone 17-22-B03) |
| 27 | Heavy chain of Ab-A-DLE-PVAΔG (based on clone 17-22-B03) |
| 28 | Heavy chain of Ab-A-AEA-PVAΔG (based on clone 17-22-B03) |
| 29 | Heavy chain of Ab-AA (based on clone 17-22-B03) |
| 30 | Heavy chain of Ab-REW (based on clone 17-22-B03) |
| 31 | Fc region including hinge region of Ab-YTE-DLE-PVAΔG |
| 32 | Fc region including hinge region of Ab-A-DLE-PVAΔG |
| 33 | Fc region including hinge region of Ab-A-AEA-PVAΔG |
| 34 | Fc region of Ab-AA (including hinge region) |
| 35 | Fc region of Ab-REW (including hinge region) |

### Examples

### Example 1: Induction of regulatory macrophages

### Methods

Frozen peripheral blood mononuclear cells (PBMC) were thawed. Cells of two individual donors were mixed in equal numbers and 2 x 10⁵ cells of the mixture were plated in 96 well plates in a total volume of 200 µL/well. Cells were incubated at 37°C/5% CO₂. After 48 h, anti-TNFα antibodies were added to reach a final concentration of 10 µg/mL. Normal human IgG (Wako Pure Chemical Industries Ltd.) was used as control. After addition of the compounds, mixed lymphocyte reactions (MLRs) were cultured for another 5 days at 37°C/5% CO₂. Afterwards, plates were washed using ice-cold phosphate buffer saline (PBS) and cells were collected using Macrophage Detachment Solution DXF (Promo Cell GmbH) and a cell scraper. CD14⁺ cells were isolated using CD14 magnetic beads (Miltenyi Biotec) and MACS system. Isolated cells were stained with an anti-CD206-PE antibody (Miltenyi Biotec, 10 µg/mL). Cells were either analyzed by FACS or by image analysis. Using image analysis, the different CD14⁺ cell populations M0, M1 and M2 (regulatory) macrophages and monocytes were differentiated by shape and CD206 expression. M0 macrophage: fried-egg shaped; M1 macrophage: spindle shaped; M2 macrophage: round shaped and CD206 positive; monocyte: round shaped and CD206 negative.

### Antibodies

Infliximab, adalimumab, certolizumab and etanercept were purchased.

The construct "Clone cA2" is an infliximab biosimilar purchased from Biorad.

"TP-IgG" is a TNFα-specific antibody having a functional Fc region. TP-IgG has a heavy chain variable domain amino acid sequence according to claim 5 of PCT/EP2017/056237 as originally filed, and a light chain variable domain amino acid sequence according to claim 6 of PCT/EP2017/056237 as originally filed (SEQ ID NO:14 of PCT/EP2017/056237, corresponding to clone 16-22-H05-sc2).

"TP-IgG Fc-sil" is identical to the above "TP-IgG", except that it contains the mutation N297A (EU numbering). The introduction of an alanine at position 297 prevents the IgG from being glycosylated, thus abrogating the Fc effector functions.

"TP-scDb" is a single chain diabody having the same heavy chain variable domain amino acid sequence and a light chain variable domain amino acid sequence as TP-IgG. "TP-scDb" lacks a functional Fc region.

"TP-scFv" is a single-chain antibody construct having the same heavy chain variable domain amino acid sequence and a light chain variable domain amino acid sequence as TP-IgG. "TP-scFv" lacks a functional Fc region.

### Results

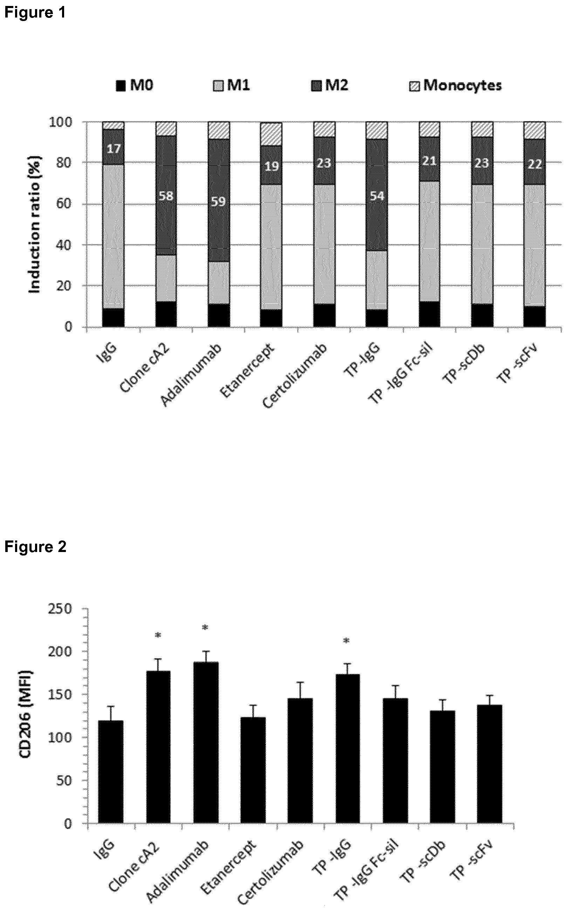

Only TNFα specific antibodies containing a functional Fc region were able to induce regulatory (M2) macrophages (Figure 1). Clone cA2 (anti-human TNF-α antibody, an infliximab biosimilar), adalimumab and TP-IgG induced M2 macrophages between 54 and 59% of the total analysed CD14⁺ cell population. The percentage of the M2 macrophages in the cultures treated with anti-TNFα antibodies without a functional Fc region were in the range of the control IgG antibody with irrelevant specificity (∼20%). Only the IgG format but not the scFv, scDb of Fc-silent IgG format increased the mean CD206 fluorescence intensity (Figure 2).

### Example 2: Wound healing assay

### Methods

Frozen PBMC were thawed. Cells of two individual donors were mixed in equal numbers and 2 x 10⁵ cells of the mixture were plated in 96 well plates in a total volume of 200 µL/well. Cells were incubated at 37°C/5% CO₂. After 48 h, anti-TNFα antibodies were added to reach a final concentration of 10 µg/mL. Normal human IgG (Wako Pure Chemical Industries Ltd.) was used as control. After addition of the compounds, mixed lymphocyte reactions (MLRs) were cultured for another 2 days at 37°C/5% CO₂. Afterwards, plates were washed using HBSS, fresh medium was added (200 µL/well) and plates were incubated for another 3 d before the cell culture supernatants (SN) were collected. SN were used both in the wound healing assay and for the determination of HGF concentration.

For the wound healing assay, colonic epithelial cells HCT-116 were cultured in parallel. On day 5 of the MLR, HCT-116 cells (2 x 10⁴ cells/well and 1 x 10⁴ cells/well) were seeded into 96-well flat-bottom tissue culture plates. On day 7, the HCT-116 cells were scratched and the size of the wounded area was determined. Medium was replaced by the collected SN from the MLR and cells were incubated for 24 h at 37°C/5% CO₂. Following the 24 h incubation, the size of the wounded area was measured again in the plates which received 2 x 10⁴ cells/well at seeding and the % closure compared to t=0 was calculated. The plates in which 1 x 10⁴ cells/well were seeded received 20 µL/well of BrdU 6 h before the end of the incubation. BrdU uptake was determined and the stimulation index (SI) calculated.

### Results

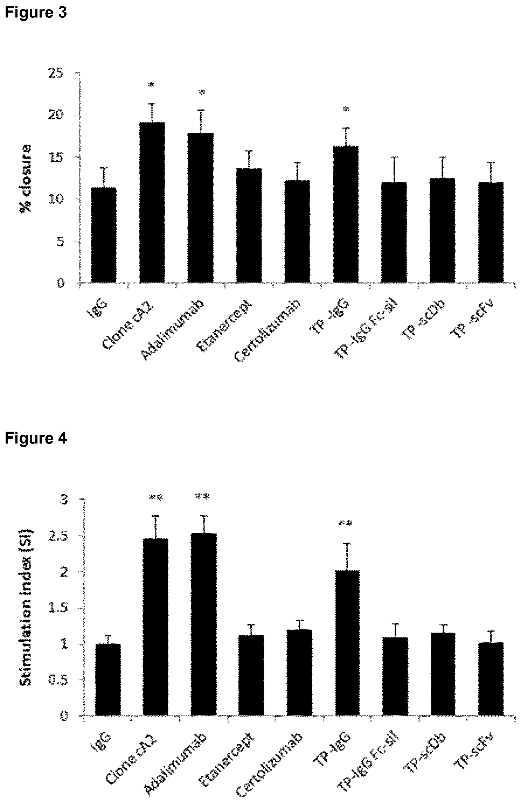

Antibodies with functional Fc region, e.g. clone cA2, adalimumab, TP-IgG, induced significant wound healing (% closure of the wounded area and SI) compared to antibodies without functional Fc region such as etanercept (Fc-fusion protein with reduced Fc functionality), certolizumab (pegylated Fab), TP-IgG Fc-silent, TP-scDb, TP-scFv (Figures 3 and 4).

### Example 3: HGF concentration

### Methods

Supernatants which were collected in the MLR and subsequently used in the wound healing assay were also assessed for the presence of HGF. HGF concentrations were determined using a HGF Quantikine ELISA kit (R&D Systems).

### Results

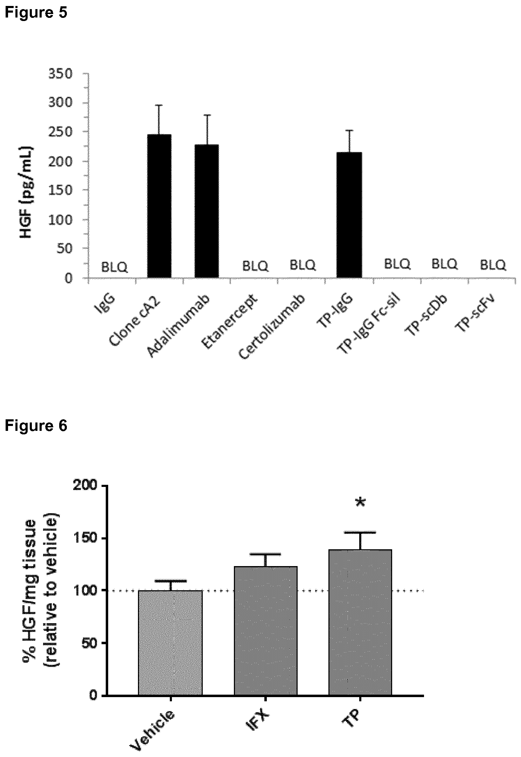

Treatment of MLRs with anti-TNFα IgG resulted in significantly increased secretion of HGF while antibody formats without functional Fc region did not induce HGF secretion (Figure 5).

### Example 4: Effect of anti-TNF-α antibodies on HGF ex vivo

### Methods

Tissue biopsies from Crohn's disease patients were placed apical so that the luminal (mucosal) side of the tissue facing upwards on a Netwell filter within a 12-well culture plate. The biopsies were then cultured in either control media or media fortified with anti-TNFα antibodies (10 µg/mL) in an incubator at 37°C and at a high O₂ atmosphere (Dame et al. (2010) In vitro Cell Dev Biol Anim 46(2):114-122) in the presence of the inflammatory stimulant SEB. SEB was used to stimulate the biopsies to increase and help normalize the inflammatory signal within the system. At approximately 18 h post-culture start, media samples were collected, protease inhibitor (Protease inhibitor Cocktail 1, Calbiochem) was added and samples were then stored at -80°C until analysis (human HGF Quantikine ELISA kit, R&D Systems).

### Results

Colonic tissue collected from Crohn's disease patients was treated with TNFα antagonists, TP or infliximab. Both antibodies induced an increase in HGF protein expression. However, only the increase after TP treatment was significant (Figure 6).

## Claims

1. An antibody comprising a TNFα-binding domain and an Fc region for use in the treatment of a wound.

2. The antibody for use according to claim 1, wherein said antibody is an IgG₁.

3. The antibody for use according to claim 1, wherein the antibody comprises (i) a V_{L} domain comprising a CDR1 region having the amino acid sequence as shown in SEQ ID NO:1, a CDR2 region having the amino acid sequence as shown in SEQ ID NO:2, and a CDR3 region having the amino acid sequence as shown in SEQ ID NO:3, and (ii) a V_{H} domain comprising a CDR1 region having the amino acid sequence as shown in SEQ ID NO:4, a CDR2 region having the amino acid sequence as shown in SEQ ID NO:5, and a CDR3 region having the amino acid sequence as shown in SEQ ID NO:6.

4. The antibody for use according to any one of the preceding claims, wherein the heavy chain of said antibody comprises an amino acid sequence selected from the group consisting of SEQ ID NO:31, SEQ ID NO:32, SEQ ID NO:33, SEQ ID NO:34 and SEQ ID NO:35.

5. The antibody for use according to claim 1, wherein said antibody is selected from the group consisting of adalimumab, infliximab and golimumab.

6. The antibody for use according to any one of the preceding claims, wherein said antibody is applied topically.

7. The antibody for use according to any one of the preceding claims, wherein said wound affects epithelial cells.

8. The antibody for use according to any one of the preceding claims, wherein said wound is associated with an ulcer in the gastrointestinal tract.

9. The antibody for use according to any one of the preceding claims, wherein said wound is associated with a stricturing or penetrating lesion of the intestinal wall.

10. The antibody for use according to any one of claims 1 to 7, wherein said wound is a surgical wound.

11. The antibody for use according to any one of claims 1 to 7, wherein said wound is associated with fibrosis, necrosis, ischemia, or vasculitis.

12. The antibody for use according to any one of the preceding claims, wherein said antibody is topically administered to the wound.

13. The antibody for use according to any one of the preceding claims, wherein said antibody is administered to a subject at a dose which is effective to induce regulatory (M2) macrophages.

14. The antibody for use according to any one of the preceding claims, wherein said antibody is administered to a subject at a dose which is effective to induce secretion of HGF in the subject.

15. The antibody for use according to any one of the preceding claims, wherein said treatment comprises administering an anti-inflammatory agent which is different from said antibody.

16. An antibody comprising a TNFα-binding domain and an Fc region for use in inducing HGF secretion.


SEQUENCE LISTING

<110>  Tillotts Pharma AG

<120>  Use of anti-TNF-alpha antibodies for treating wounds

<130>  PEP02787TIL

<160>  35

<170>  PatentIn version 3.5

<210>  1
<211>  11
<212>  PRT
<213>  Artificial Sequence

<220>
<223>  CDR L1 of clone 16-22-H05

<400>  1

Gln Ala Ser Gln Ser Ile Phe Ser Gly Leu Ala
1               5                   10


<210>  2
<211>  7
<212>  PRT
<213>  Artificial Sequence

<220>
<223>  CDR L2 of clone 16-22-H05

<400>  2

Gly Ala Ser Lys Leu Ala Ser
1               5


<210>  3
<211>  15
<212>  PRT
<213>  Artificial Sequence

<220>
<223>  CDR L3 of clone 16-22-H05

<400>  3

Gln Ser Tyr Tyr Tyr Ser Ser Ser Ser Ser Asp Gly Ser Tyr Ala
1               5                   10                  15


<210>  4
<211>  10
<212>  PRT
<213>  Artificial Sequence

<220>
<223>  CDR H1 of clone 16-22-H05

<400>  4

Gly Ile Asp Phe Asn Asn Tyr Gly Ile Gly
1               5                   10

<210>  5
<211>  17
<212>  PRT
<213>  Artificial Sequence

<220>
<223>  CDR H2 of clone 16-22-H05

<400>  5

Tyr Ile Tyr Pro Gly Phe Ala Ile Thr Asn Phe Ala Asn Ser Val Lys
1               5                   10                  15

Gly

<210>  6
<211>  13
<212>  PRT
<213>  Artificial Sequence

<220>
<223>  CDR H3 of clone 16-22-H05

<400>  6

Asp Pro Val Tyr Ala Thr Ser Ser Gly Tyr Phe Asp Leu
1               5                   10

<210>  7
<211>  122
<212>  PRT
<213>  Artificial Sequence

<220>
<223>  VH of humanized IgG of clone 16-22-H05

<400>  7

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Asn Asn Tyr
            20                  25                  30

Gly Ile Gly Trp Val Arg Gln Ala Pro Gly Lys Gly Leu Glu Trp Ile
        35                  40                  45

Thr Tyr Ile Tyr Pro Gly Phe Ala Ile Thr Asn Phe Ala Asn Ser Val
    50                  55                  60

Lys Gly Arg Phe Thr Ile Ser Ser Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Arg Asp Pro Val Tyr Ala Thr Ser Ser Gly Tyr Phe Asp Leu Trp
            100                 105                 110

Gly Gln Gly Thr Leu Val Thr Val Ser Ser
        115                 120

<210>  8
<211>  114
<212>  PRT
<213>  Artificial Sequence

<220>
<223>  VL of humanized IgG of clone 16-22-H05

<400>  8

Asp Ile Gln Met Thr Gln Ser Pro Ser Ser Leu Ser Ala Ser Val Gly
1               5                   10                  15

Asp Arg Val Thr Ile Lys Cys Gln Ala Ser Gln Ser Ile Phe Ser Gly
            20                  25                  30

Leu Ala Trp Tyr Gln Gln Lys Pro Gly Lys Ala Pro Lys Leu Leu Ile
        35                  40                  45

Tyr Gly Ala Ser Lys Leu Ala Ser Gly Val Pro Ser Arg Phe Ser Gly
    50                  55                  60

Ser Gly Ser Gly Thr Glu Phe Thr Leu Thr Ile Ser Gly Leu Gln Pro
65                  70                  75                  80

Ala Asp Phe Ala Thr Tyr Tyr Cys Gln Ser Tyr Tyr Tyr Ser Ser Ser
                85                  90                  95

Ser Ser Asp Gly Ser Tyr Ala Phe Gly Gly Gly Thr Lys Leu Thr Val
            100                 105                 110

Leu Gly

<210>  9
<211>  221
<212>  PRT
<213>  Artificial Sequence

<220>
<223>  Light chain of humanized IgG of clone 16-22-H05

<400>  9

Asp Ile Gln Met Thr Gln Ser Pro Ser Ser Leu Ser Ala Ser Val Gly
1               5                   10                  15

Asp Arg Val Thr Ile Lys Cys Gln Ala Ser Gln Ser Ile Phe Ser Gly
            20                  25                  30

Leu Ala Trp Tyr Gln Gln Lys Pro Gly Lys Ala Pro Lys Leu Leu Ile
        35                  40                  45

Tyr Gly Ala Ser Lys Leu Ala Ser Gly Val Pro Ser Arg Phe Ser Gly
    50                  55                  60

Ser Gly Ser Gly Thr Glu Phe Thr Leu Thr Ile Ser Gly Leu Gln Pro
65                  70                  75                  80

Ala Asp Phe Ala Thr Tyr Tyr Cys Gln Ser Tyr Tyr Tyr Ser Ser Ser
                85                  90                  95

Ser Ser Asp Gly Ser Tyr Ala Phe Gly Gly Gly Thr Lys Leu Thr Val
            100                 105                 110

Leu Gly Arg Thr Val Ala Ala Pro Ser Val Phe Ile Phe Pro Pro Ser
        115                 120                 125

Asp Glu Gln Leu Lys Ser Gly Thr Ala Ser Val Val Cys Leu Leu Asn
    130                 135                 140

Asn Phe Tyr Pro Arg Glu Ala Lys Val Gln Trp Lys Val Asp Asn Ala
145                 150                 155                 160

Leu Gln Ser Gly Asn Ser Gln Glu Ser Val Thr Glu Gln Asp Ser Lys
                165                 170                 175

Asp Ser Thr Tyr Ser Leu Ser Ser Thr Leu Thr Leu Ser Lys Ala Asp
            180                 185                 190

Tyr Glu Lys His Lys Val Tyr Ala Cys Glu Val Thr His Gln Gly Leu
        195                 200                 205

Ser Ser Pro Val Thr Lys Ser Phe Asn Arg Gly Glu Cys
    210                 215                 220

<210>  10
<211>  451

<212>  PRT
<213>  Artificial Sequence

<220>
<223>  Heavy chain of Ab-YTE-DLE-PVAdeltaG (based on clone 16-22-H05)

<400>  10

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Asn Asn Tyr
            20                  25                  30

Gly Ile Gly Trp Val Arg Gln Ala Pro Gly Lys Gly Leu Glu Trp Ile
        35                  40                  45

Thr Tyr Ile Tyr Pro Gly Phe Ala Ile Thr Asn Phe Ala Asn Ser Val
    50                  55                  60

Lys Gly Arg Phe Thr Ile Ser Ser Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Arg Asp Pro Val Tyr Ala Thr Ser Ser Gly Tyr Phe Asp Leu Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Pro Val Ala
225                 230                 235                 240

Gly Pro Asp Val Phe Leu Phe Pro Pro Lys Pro Lys Asp Thr Leu Tyr
                245                 250                 255

Ile Thr Arg Glu Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Leu Pro Glu
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys
    450

<210>  11
<211>  451

<212>  PRT
<213>  Artificial Sequence

<220>
<223>  Heavy chain of Ab-A-DLE-PVAdeltaG (based on clone 16-22-H05)

<400>  11

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Asn Asn Tyr
            20                  25                  30

Gly Ile Gly Trp Val Arg Gln Ala Pro Gly Lys Gly Leu Glu Trp Ile
        35                  40                  45

Thr Tyr Ile Tyr Pro Gly Phe Ala Ile Thr Asn Phe Ala Asn Ser Val
    50                  55                  60

Lys Gly Arg Phe Thr Ile Ser Ser Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Arg Asp Pro Val Tyr Ala Thr Ser Ser Gly Tyr Phe Asp Leu Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Pro Val Ala
225                 230                 235                 240

Gly Pro Asp Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Leu Pro Glu
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Ala His Tyr Thr Gln Lys Ser Leu Ser Leu Ser
        435                 440                 445

Pro Gly Lys
    450

<210>  12
<211>  451

<212>  PRT
<213>  Artificial Sequence

<220>
<223>  Heavy chain of Ab-A-AEA-PVAdeltaG (based on clone 16-22-H05)

<400>  12

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Asn Asn Tyr
            20                  25                  30

Gly Ile Gly Trp Val Arg Gln Ala Pro Gly Lys Gly Leu Glu Trp Ile
        35                  40                  45

Thr Tyr Ile Tyr Pro Gly Phe Ala Ile Thr Asn Phe Ala Asn Ser Val
    50                  55                  60

Lys Gly Arg Phe Thr Ile Ser Ser Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Arg Asp Pro Val Tyr Ala Thr Ser Ser Gly Tyr Phe Asp Leu Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Pro Val Ala
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Ala Ala Leu Pro Ala Pro Glu
                325                 330                 335

Ala Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Ala His Tyr Thr Gln Lys Ser Leu Ser Leu Ser
        435                 440                 445

Pro Gly Lys
    450

<210>  13
<211>  452

<212>  PRT
<213>  Artificial Sequence

<220>
<223>  Heavy chain of Ab-AA (based on clone 16-22-H05)

<400>  13

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Asn Asn Tyr
            20                  25                  30

Gly Ile Gly Trp Val Arg Gln Ala Pro Gly Lys Gly Leu Glu Trp Ile
        35                  40                  45

Thr Tyr Ile Tyr Pro Gly Phe Ala Ile Thr Asn Phe Ala Asn Ser Val
    50                  55                  60

Lys Gly Arg Phe Thr Ile Ser Ser Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Arg Asp Pro Val Tyr Ala Thr Ser Ser Gly Tyr Phe Asp Leu Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Glu Glu Met Thr Lys Asn Gln Val
        355                 360                 365

Ser Leu Thr Cys Leu Val Lys Gly Phe Tyr Pro Ser Asp Ile Ala Val
    370                 375                 380

Ala Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Ala His Tyr Thr Gln Lys Ser Leu Ser Leu
        435                 440                 445

Ser Pro Gly Lys
    450

<210>  14
<211>  452

<212>  PRT
<213>  Artificial Sequence

<220>
<223>  Heavy chain of Ab-REW (based on clone 16-22-H05)

<400>  14

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Asn Asn Tyr
            20                  25                  30

Gly Ile Gly Trp Val Arg Gln Ala Pro Gly Lys Gly Leu Glu Trp Ile
        35                  40                  45

Thr Tyr Ile Tyr Pro Gly Phe Ala Ile Thr Asn Phe Ala Asn Ser Val
    50                  55                  60

Lys Gly Arg Phe Thr Ile Ser Ser Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Arg Asp Pro Val Tyr Ala Thr Ser Ser Gly Tyr Phe Asp Leu Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Arg Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Glu Glu Met Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Glu His Glu Ala Leu His Trp His Tyr Thr Gln Lys Ser Leu Ser Leu
        435                 440                 445

Ser Pro Gly Lys
    450

<210>  15
<211>  11

<212>  PRT
<213>  Artificial Sequence

<220>
<223>  CDR L1 of clone 17-22-B03

<400>  15

Gln Ala Ser Gln Ser Ile Ser Ser Tyr Leu Ala
1               5                   10


<210>  16
<211>  7
<212>  PRT
<213>  Artificial Sequence

<220>
<223>  CDR L2 of clone 17-22-B03

<400>  16

Lys Ala Ser Thr Leu Ala Ser
1               5


<210>  17
<211>  13
<212>  PRT
<213>  Artificial Sequence

<220>
<223>  CDR L3 of clone 17-22-B03

<400>  17

Gln Cys Thr Tyr Tyr Glu Pro Ser Tyr Val Phe Arg Ala
1               5                   10


<210>  18
<211>  11
<212>  PRT
<213>  Artificial Sequence

<220>
<223>  CDR H1 of clone 17-22-B03

<400>  18

Gly Ile Asp Phe Ser Ala Gly Tyr Asp Met Cys
1               5                   10


<210>  19
<211>  17
<212>  PRT
<213>  Artificial Sequence

<220>
<223>  CDR H2 of clone 17-22-B03

<400>  19

Cys Ile Asp Ser Arg Arg Glu Glu Ser Asp Tyr Ala Ser Trp Ala Lys
1               5                   10                  15

Gly

<210>  20
<211>  14
<212>  PRT
<213>  Artificial Sequence

<220>
<223>  CDR H3 of clone 17-22-B03

<400>  20

Gly Gly Tyr Gly Gly Asp Gly Val Asp Gly Ala Phe Asp Pro
1               5                   10

<210>  21
<211>  124
<212>  PRT
<213>  Artificial Sequence

<220>
<223>  VH of humanized IgG of clone 17-22-B03

<400>  21

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Ser Ala Gly
            20                  25                  30

Tyr Asp Met Cys Trp Val Arg Gln Ala Pro Gly Lys Gly Leu Glu Trp
        35                  40                  45

Ile Ala Cys Ile Asp Ser Arg Arg Glu Glu Ser Asp Tyr Ala Ser Trp
    50                  55                  60

Ala Lys Gly Arg Phe Thr Ile Ser Lys Asp Ser Ser Lys Asn Thr Val
65                  70                  75                  80

Tyr Leu Gln Met Asn Ser Leu Arg Ala Glu Asp Thr Ala Val Tyr Tyr
                85                  90                  95

Cys Ala Arg Gly Gly Tyr Gly Gly Asp Gly Val Asp Gly Ala Phe Asp
            100                 105                 110

Pro Trp Gly Gln Gly Thr Leu Val Thr Val Ser Ser
        115                 120

<210>  22
<211>  112
<212>  PRT
<213>  Artificial Sequence

<220>
<223>  VL of humanized IgG of clone 17-22-B03 -(sc08)

<400>  22

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Ser Ser Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Cys Thr Tyr Tyr Glu Pro Ser
                85                  90                  95

Tyr Val Phe Arg Ala Phe Gly Gly Gly Thr Lys Leu Thr Val Leu Gly
            100                 105                 110


<210>  23
<211>  112
<212>  PRT
<213>  Artificial Sequence

<220>
<223>  VL of humanized IgG of clone 17-22-B03 -(sc02)

<400>  23

Asp Ile Gln Met Thr Gln Ser Pro Ser Ser Leu Ser Ala Ser Val Gly
1               5                   10                  15

Asp Arg Val Thr Ile Lys Cys Gln Ala Ser Gln Ser Ile Ser Ser Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly

50                  55                  60

Ser Gly Ser Gly Thr Glu Phe Thr Leu Thr Ile Ser Asp Leu Glu Pro
65                  70                  75                  80

Ala Asp Phe Ala Thr Tyr Tyr Cys Gln Cys Thr Tyr Tyr Glu Pro Ser
                85                  90                  95

Tyr Val Phe Arg Ala Phe Gly Gly Gly Thr Lys Leu Thr Val Leu Gly
            100                 105                 110

<210>  24
<211>  219
<212>  PRT
<213>  Artificial Sequence

<220>
<223>  Light chain of humanized IgG of clone 17-22-B03 -(sc08)

<400>  24

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Ser Ser Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Cys Thr Tyr Tyr Glu Pro Ser
                85                  90                  95

Tyr Val Phe Arg Ala Phe Gly Gly Gly Thr Lys Leu Thr Val Leu Gly
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  25
<211>  219
<212>  PRT
<213>  Artificial Sequence

<220>
<223>  Light chain of humanized IgG of clone 17-22-B03 -(sc02)

<400>  25

Asp Ile Gln Met Thr Gln Ser Pro Ser Ser Leu Ser Ala Ser Val Gly
1               5                   10                  15

Asp Arg Val Thr Ile Lys Cys Gln Ala Ser Gln Ser Ile Ser Ser Tyr
            20                  25                  30

Leu Ala Trp Tyr Gln Gln Lys Pro Gly Lys Ala Pro Lys Leu Leu Ile
        35                  40                  45

Tyr Lys Ala Ser Thr Leu Ala Ser Gly Val Pro Ser Arg Phe Ser Gly
    50                  55                  60

Ser Gly Ser Gly Thr Glu Phe Thr Leu Thr Ile Ser Asp Leu Glu Pro
65                  70                  75                  80

Ala Asp Phe Ala Thr Tyr Tyr Cys Gln Cys Thr Tyr Tyr Glu Pro Ser
                85                  90                  95

Tyr Val Phe Arg Ala Phe Gly Gly Gly Thr Lys Leu Thr Val Leu Gly
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  26
<211>  453
<212>  PRT
<213>  Artificial Sequence

<220>
<223>  Heavy chain of Ab-YTE-DLE-PVAdeltaG (based on clone 17-22-B03)

<400>  26

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Ser Ala Gly
            20                  25                  30

Tyr Asp Met Cys Trp Val Arg Gln Ala Pro Gly Lys Gly Leu Glu Trp
        35                  40                  45

Ile Ala Cys Ile Asp Ser Arg Arg Glu Glu Ser Asp Tyr Ala Ser Trp
    50                  55                  60

Ala Lys Gly Arg Phe Thr Ile Ser Lys Asp Ser Ser Lys Asn Thr Val
65                  70                  75                  80

Tyr Leu Gln Met Asn Ser Leu Arg Ala Glu Asp Thr Ala Val Tyr Tyr
                85                  90                  95

Cys Ala Arg Gly Gly Tyr Gly Gly Asp Gly Val Asp Gly Ala Phe Asp
            100                 105                 110

Pro Trp Gly Gln Gly Thr Leu Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Pro
225                 230                 235                 240

Val Ala Gly Pro Asp Val Phe Leu Phe Pro Pro Lys Pro Lys Asp Thr
                245                 250                 255

Leu Tyr Ile Thr Arg Glu Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Leu
                325                 330                 335

Pro Glu Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210>  27
<211>  453
<212>  PRT
<213>  Artificial Sequence

<220>
<223>  Heavy chain of Ab-A-DLE-PVAdeltaG (based on clone 17-22-B03)

<400>  27

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Ser Ala Gly
            20                  25                  30

Tyr Asp Met Cys Trp Val Arg Gln Ala Pro Gly Lys Gly Leu Glu Trp
        35                  40                  45

Ile Ala Cys Ile Asp Ser Arg Arg Glu Glu Ser Asp Tyr Ala Ser Trp
    50                  55                  60

Ala Lys Gly Arg Phe Thr Ile Ser Lys Asp Ser Ser Lys Asn Thr Val
65                  70                  75                  80

Tyr Leu Gln Met Asn Ser Leu Arg Ala Glu Asp Thr Ala Val Tyr Tyr
                85                  90                  95

Cys Ala Arg Gly Gly Tyr Gly Gly Asp Gly Val Asp Gly Ala Phe Asp
            100                 105                 110

Pro Trp Gly Gln Gly Thr Leu Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Pro
225                 230                 235                 240

Val Ala Gly Pro Asp Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Leu
                325                 330                 335

Pro Glu Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Ala His Tyr Thr Gln Lys Ser Leu Ser
        435                 440                 445

Leu Ser Pro Gly Lys
    450

<210>  28
<211>  453
<212>  PRT
<213>  Artificial Sequence

<220>
<223>  Heavy chain of Ab-A-AEA-PVAdeltaG (based on clone 17-22-B03)

<400>  28

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Ser Ala Gly
            20                  25                  30

Tyr Asp Met Cys Trp Val Arg Gln Ala Pro Gly Lys Gly Leu Glu Trp
        35                  40                  45

Ile Ala Cys Ile Asp Ser Arg Arg Glu Glu Ser Asp Tyr Ala Ser Trp
    50                  55                  60

Ala Lys Gly Arg Phe Thr Ile Ser Lys Asp Ser Ser Lys Asn Thr Val
65                  70                  75                  80

Tyr Leu Gln Met Asn Ser Leu Arg Ala Glu Asp Thr Ala Val Tyr Tyr
                85                  90                  95

Cys Ala Arg Gly Gly Tyr Gly Gly Asp Gly Val Asp Gly Ala Phe Asp
            100                 105                 110

Pro Trp Gly Gln Gly Thr Leu Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Pro
225                 230                 235                 240

Val Ala Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Ala Ala Leu Pro Ala
                325                 330                 335

Pro Glu Ala Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Ala His Tyr Thr Gln Lys Ser Leu Ser
        435                 440                 445

Leu Ser Pro Gly Lys
    450

<210>  29
<211>  454
<212>  PRT
<213>  Artificial Sequence

<220>
<223>  Heavy chain of Ab-AA (based on clone 17-22-B03)

<400>  29

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Ser Ala Gly
            20                  25                  30

Tyr Asp Met Cys Trp Val Arg Gln Ala Pro Gly Lys Gly Leu Glu Trp
        35                  40                  45

Ile Ala Cys Ile Asp Ser Arg Arg Glu Glu Ser Asp Tyr Ala Ser Trp
    50                  55                  60

Ala Lys Gly Arg Phe Thr Ile Ser Lys Asp Ser Ser Lys Asn Thr Val
65                  70                  75                  80

Tyr Leu Gln Met Asn Ser Leu Arg Ala Glu Asp Thr Ala Val Tyr Tyr
                85                  90                  95

Cys Ala Arg Gly Gly Tyr Gly Gly Asp Gly Val Asp Gly Ala Phe Asp
            100                 105                 110

Pro Trp Gly Gln Gly Thr Leu Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Glu
225                 230                 235                 240

Leu Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser His Glu Asp Pro Glu Val Lys Phe Asn Trp Tyr Val Asp Gly
        275                 280                 285

Val Glu Val His Asn Ala Lys Thr Lys Pro Arg Glu Glu Gln Tyr Asn
    290                 295                 300

Ser Thr Tyr Arg Val Val Ser Val Leu Thr Val Leu His Gln Asp Trp
305                 310                 315                 320

Leu Asn Gly Lys Glu Tyr Lys Cys Lys Val Ser Asn Lys Ala Leu Pro
                325                 330                 335

Ala Pro Ile Glu Lys Thr Ile Ser Lys Ala Lys Gly Gln Pro Arg Glu
            340                 345                 350

Pro Gln Val Tyr Thr Leu Pro Pro Ser Arg Glu Glu Met Thr Lys Asn
        355                 360                 365

Gln Val Ser Leu Thr Cys Leu Val Lys Gly Phe Tyr Pro Ser Asp Ile
    370                 375                 380

Ala Val Ala Trp Glu Ser Asn Gly Gln Pro Glu Asn Asn Tyr Lys Thr
385                 390                 395                 400

Thr Pro Pro Val Leu Asp Ser Asp Gly Ser Phe Phe Leu Tyr Ser Lys
                405                 410                 415

Leu Thr Val Asp Lys Ser Arg Trp Gln Gln Gly Asn Val Phe Ser Cys
            420                 425                 430

Ser Val Met His Glu Ala Leu His Ala His Tyr Thr Gln Lys Ser Leu
        435                 440                 445

Ser Leu Ser Pro Gly Lys
    450

<210>  30
<211>  454
<212>  PRT
<213>  Artificial Sequence

<220>
<223>  Heavy chain of Ab-REW (based on clone 17-22-B03)

<400>  30

Glu Val Gln Leu Val Glu Ser Gly Gly Gly Leu Val Gln Pro Gly Gly
1               5                   10                  15

Ser Leu Arg Leu Ser Cys Lys Ala Ser Gly Ile Asp Phe Ser Ala Gly
            20                  25                  30

Tyr Asp Met Cys Trp Val Arg Gln Ala Pro Gly Lys Gly Leu Glu Trp
        35                  40                  45

Ile Ala Cys Ile Asp Ser Arg Arg Glu Glu Ser Asp Tyr Ala Ser Trp
    50                  55                  60

Ala Lys Gly Arg Phe Thr Ile Ser Lys Asp Ser Ser Lys Asn Thr Val
65                  70                  75                  80

Tyr Leu Gln Met Asn Ser Leu Arg Ala Glu Asp Thr Ala Val Tyr Tyr
                85                  90                  95

Cys Ala Arg Gly Gly Tyr Gly Gly Asp Gly Val Asp Gly Ala Phe Asp
            100                 105                 110

Pro Trp Gly Gln Gly Thr Leu Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Glu
225                 230                 235                 240

Leu Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser His Glu Asp Pro Glu Val Lys Phe Asn Trp Tyr Val Asp Gly
        275                 280                 285

Val Glu Val His Asn Ala Lys Thr Lys Pro Arg Glu Glu Gln Tyr Asn
    290                 295                 300

Ser Thr Tyr Arg Val Val Ser Val Leu Thr Val Leu His Arg Asp Trp
305                 310                 315                 320

Leu Asn Gly Lys Glu Tyr Lys Cys Lys Val Ser Asn Lys Ala Leu Pro
                325                 330                 335

Ala Pro Ile Glu Lys Thr Ile Ser Lys Ala Lys Gly Gln Pro Arg Glu
            340                 345                 350

Pro Gln Val Tyr Thr Leu Pro Pro Ser Arg Glu Glu Met Thr Lys Asn
        355                 360                 365

Gln Val Ser Leu Thr Cys Leu Val Lys Gly Phe Tyr Pro Ser Asp Ile
    370                 375                 380

Ala Val Glu Trp Glu Ser Asn Gly Gln Pro Glu Asn Asn Tyr Lys Thr
385                 390                 395                 400

Thr Pro Pro Val Leu Asp Ser Asp Gly Ser Phe Phe Leu Tyr Ser Lys
                405                 410                 415

Leu Thr Val Asp Lys Ser Arg Trp Gln Gln Gly Asn Val Phe Ser Cys
            420                 425                 430

Ser Val Glu His Glu Ala Leu His Trp His Tyr Thr Gln Lys Ser Leu
        435                 440                 445

Ser Leu Ser Pro Gly Lys
    450

<210>  31
<211>  231
<212>  PRT
<213>  Artificial Sequence

<220>
<223>  Fc region of Ab-YTE-DLE-PVAdeltaG (including hinge region)

<400>  31

Glu Pro Lys Ser Cys Asp Lys Thr His Thr Cys Pro Pro Cys Pro Ala
1               5                   10                  15

Pro Pro Val Ala Gly Pro Asp Val Phe Leu Phe Pro Pro Lys Pro Lys
            20                  25                  30

Asp Thr Leu Tyr Ile Thr Arg Glu Pro Glu Val Thr Cys Val Val Val
        35                  40                  45

Asp Val Ser His Glu Asp Pro Glu Val Lys Phe Asn Trp Tyr Val Asp
    50                  55                  60

Gly Val Glu Val His Asn Ala Lys Thr Lys Pro Arg Glu Glu Gln Tyr
65                  70                  75                  80

Asn Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Lys Ala Leu
            100                 105                 110

Pro Leu Pro Glu Glu Lys Thr Ile Ser Lys Ala Lys Gly Gln Pro Arg
        115                 120                 125

Glu Pro Gln Val Tyr Thr Leu Pro Pro Ser Arg Glu Glu Met Thr Lys
    130                 135                 140

Asn Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Thr Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Asn His Tyr Thr Gln Lys Ser
    210                 215                 220

Leu Ser Leu Ser Pro Gly Lys
225                 230

<210>  32
<211>  231
<212>  PRT
<213>  Artificial Sequence

<220>
<223>  Fc region of Ab-A-DLE-PVAdeltaG  (including hinge region)

<400>  32

Glu Pro Lys Ser Cys Asp Lys Thr His Thr Cys Pro Pro Cys Pro Ala
1               5                   10                  15

Pro Pro Val Ala Gly Pro Asp Val Phe Leu Phe Pro Pro Lys Pro Lys
            20                  25                  30

Asp Thr Leu Met Ile Ser Arg Thr Pro Glu Val Thr Cys Val Val Val
        35                  40                  45

Asp Val Ser His Glu Asp Pro Glu Val Lys Phe Asn Trp Tyr Val Asp
    50                  55                  60

Gly Val Glu Val His Asn Ala Lys Thr Lys Pro Arg Glu Glu Gln Tyr
65                  70                  75                  80

Asn Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Lys Ala Leu

            100                 105                 110

Pro Leu Pro Glu Glu Lys Thr Ile Ser Lys Ala Lys Gly Gln Pro Arg
        115                 120                 125

Glu Pro Gln Val Tyr Thr Leu Pro Pro Ser Arg Glu Glu Met Thr Lys
    130                 135                 140

Asn Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Thr Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Ala His Tyr Thr Gln Lys Ser
    210                 215                 220

Leu Ser Leu Ser Pro Gly Lys
225                 230

<210> 33
<211> 231
<212> PRT
<213> Artificial Sequence

<220>
<223> Fc region of Ab-A-AEA-PVAdeltaG (including hinge region)

<400> 33

Glu Pro Lys Ser Cys Asp Lys Thr His Thr Cys Pro Pro Cys Pro Ala
1               5                   10                  15

Pro Pro Val Ala Gly Pro Ser Val Phe Leu Phe Pro Pro Lys Pro Lys
            20                  25                  30

Asp Thr Leu Met Ile Ser Arg Thr Pro Glu Val Thr Cys Val Val Val
        35                  40                  45

Asp Val Ser His Glu Asp Pro Glu Val Lys Phe Asn Trp Tyr Val Asp
    50                  55                  60

Gly Val Glu Val His Asn Ala Lys Thr Lys Pro Arg Glu Glu Gln Tyr
65                  70                  75                  80

Asn Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Ala Ala Leu
            100                 105                 110

Pro Ala Pro Glu Ala Lys Thr Ile Ser Lys Ala Lys Gly Gln Pro Arg
        115                 120                 125

Glu Pro Gln Val Tyr Thr Leu Pro Pro Ser Arg Glu Glu Met Thr Lys
    130                 135                 140

Asn Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Thr Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Ala His Tyr Thr Gln Lys Ser
    210                 215                 220

Leu Ser Leu Ser Pro Gly Lys
225                 230

<210>  34
<211>  232
<212>  PRT
<213>  Artificial Sequence

<220>
<223>  Fc region of Ab-AA (including hinge region)

<400>  34

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Glu Glu Met Thr
    130                 135                 140

Lys Asn Gln Val Ser Leu Thr Cys Leu Val Lys Gly Phe Tyr Pro Ser
145                 150                 155                 160

Asp Ile Ala Val Ala Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Ala His Tyr Thr Gln Lys
    210                 215                 220

Ser Leu Ser Leu Ser Pro Gly Lys
225                 230

<210>  35
<211>  232
<212>  PRT
<213>  Artificial Sequence

<220>
<223>  Fc region of Ab-REW (including hinge region)

<400>  35

Glu Pro Lys Ser Cys Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Arg
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Glu Glu Met Thr
    130                 135                 140

Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Glu His Glu Ala Leu His Trp His Tyr Thr Gln Lys
    210                 215                 220

Ser Leu Ser Leu Ser Pro Gly Lys
225                 230